# FASCICULE DE BREVET EUROPEEN

(11) **EP 0 857 472 B1**
(45) Date de publication et mention de la délivrance du brevet: **08.05.2002**
(21) Numéro de dépôt: 98420003.0
(22) Date de dépôt: 14.01.1998
(51) Int. Cl.: A61F 2/38

(54) **Prothèse totale de coude**
Totale Ellbogenprothese
Total elbow prosthesis

(30) Priorité: 23.01.1997 FR 9700909
(43) Date de publication de la demande: 12.08.1998
(73) Titulaire: Tornier SA, 38330 Saint Ismier (FR)
(72) Inventeur: Judet, Thierry, 92410 Ville D'Avray (FR); Tornier, Alain, 38330 Saint Ismier (FR)
(74) Mandataire: Schmitt, John

(56) Documents cités:
- DE-A- 4 331 282
- FR-A- 2 663 838
- US-A- 4 242 758

## Description

La présente invention a trait à une prothèse totale du coude du genre comprenant un premier élément huméral, un second élément cubital, et un troisième élément radial.

On connaît d'après le brevet US 4 242 758 des prothèses de ce type comportant ces trois éléments mais dont le troisième élément radial est solidaire de sa tige, et fixe par rapport à l'os, empêchant toute possibilité de liberté en rotation autour de ses axes.

De telles prothèses comportent certains inconvénients en particulier des risques d'instabilité, de douleurs et de descellements.

On connaît d'après le brevet DE 43 31282 une prothèse radiale du coude comportant une partie monobloc destinée à être fixée dans le radius et une partie modulaire qui constitue la surface articulaire de la prothèse composée d'un insert et d'une cupule qui peuvent bouger l'un par rapport à l'autre.

La prothèse totale de coude suivant la présente invention a pour objet de rétablir l'anatomie du coude, en proposant un élément radial destiné à s'articuler avec la surface radiale et la surface externe cubitale et dont la tige est ancrée en respectant l'orientation naturelle du radius, tout en maintenant la tête radiale dans la bonne orientation sur l'humérus.

La prothèse de coude suivant la présente invention comprend un élément radial qui comporte des moyens pour réaliser les articulations avec la surface articulaire radiale de l'élément huméral et la surface articulaire externe de l'élément cubital.

De plus, la prothèse totale de coude comporte un élément radial muni d'une tige d'ancrage pourvue d'un col solidaire d'une boule sur laquelle vient s'articuler une tête cylindrique dont la face supérieure présente un profil concave qui coopère avec la surface articulaire radiale de l'élément huméral, tandis que le pourtour cylindrique de la tête coopère avec la surface articulaire exteme de l'élément cubital.

La prothèse totale de coude suivant la présente invention comprend, un premier élément huméral muni d'une surface articulaire épicondylaire, un second élément cubital pourvu d'une surface articulaire et d'un élément radial comportant une tige d'ancrage pourvue d'un col qui est incliné d'un angle β compris entre 0 et 30° par rapport à l'axe de ladite tige, ledit col étant solidaire d'une boule sur laquelle vient s'articuler une tête cylindrique comprenant une face supérieure à profil concave qui coopère avec la surface épicondylaire de l'élément huméral et un pourtour cylindrique coopérant avec une surface articulaire externe prévue sur le flanc externe d'une embase de l'élément cubital.

La prothèse de coude suivant la présente invention comporte une tête articulaire qui reçoit un cerclage métallique propre à coopérer avec la surface articulaire de l'élément cubital réalisé en polyéthylène.

La prothèse de coude suivant la présente invention comporte une tête articulaire comprenant un logement sphérique muni d'une partie conique pour recevoir la boule afin de permettre à ladite tête un débattement angulaire.

La prothèse de coude suivant la présente invention comporte un élément radial comprenant une tête articulaire pourvue d'un corps en polyéthylène enveloppée d'une cupule métallique.

La prothèse de coude suivant la présente invention comporte une tête articulaire qui est complètement libre suivant ses trois degrés de liberté en rotation par rapport au centre de la boule.

La prothèse de coude suivant la présente invention comporte une tête articulaire et une boule de l'élément radial qui sont réalisés en céramique.

La description qui va suivre en regard des dessins annexés, donnés à titre d'exemple non limitatif, permettra de mieux comprendre l'invention, les caractéristiques qu'elle présente, les avantages qu'elle est susceptible de procurer.

La figure 1 est une vue d'un bras droit représentant la prothèse de coude suivant l'invention dans le plan frontal anatomique, bras tendu.

La figure 2 est une vue montrant l'élément radial de la prothèse de coude suivant la présente invention, muni de sa tête réalisée par exemple en plastique.

La figure 3 est une vue illustrant une première variante de la prothèse de coude.

La figure 4 est une vue montrant une articulation de coude munie uniquement de l'élément radial de la prothèse de coudé suivant la présente invention.

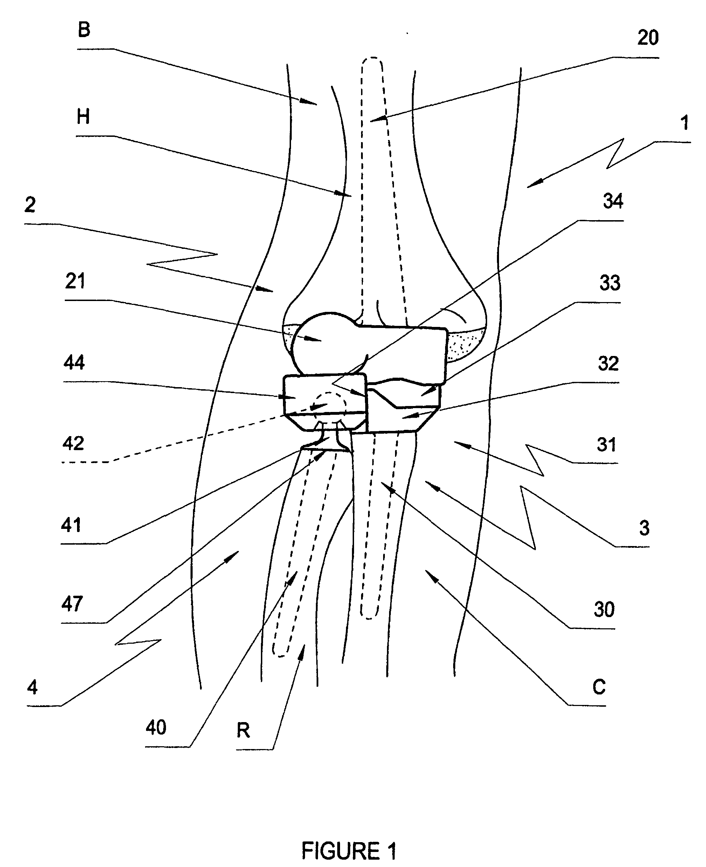

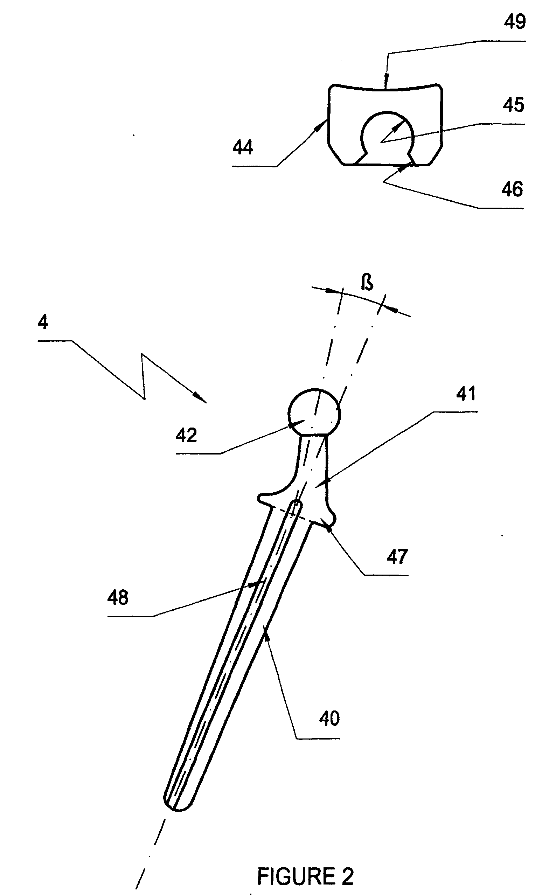

On a représenté en figures 1 et 2 une prothèse totale de coude 1 constituée d'un élément huméral 2 prévue pour s'ancrer dans,l'humérus H, d'un élément cubital 3 solidaire du cubitus C et d'un élément radial 4 fixé dans le radius R d'un bras droit B.

L'élément huméral 1 comprend une partie articulaire métallique reproduisant les portions d'articulation anatomiques destinées à entrer en contact avec la tête radiale du côté externe et le cubitus du côté interne.

L'élément huméral comporte une tige d'ancrage 20 solidaire d'une partie épicondylaire 21 reproduisant la surface articulaire de l'humérus.

L'élément cubital 3 comprend une tige d'ancrage 30 destinée à l'ancrage dans le cubitus C, une surface articulaire 31 coopérant avec la partie épicondylaire 21 de la pièce humérale 2.

La surface articulaire 31 est, par exemple, composée d'une embase métallique 32 solidaire de la tige 30, sur laquelle vient se fixer un plateau en polyéthylène 33 venant en regard de la surface articulaire correspondante de la partie épicondylaire 21.

L'embase métallique 32 présente sur son flanc externe, dans un plan sensiblement parallèle à l'axe de la tige 30 de l'élément cubital 3, une surface articulaire 34, à profil sensiblement concave.

L'élément radial 4 représenté en figure 1 et 2 présente une tige 40 destinée à s'ancrer dans le radius R et surmontée par un col 41 solidaire d'une boule 42 sur laquelle s'emboîte une tête articulaire 44 de forme généralement cylindrique ou conique.

La tête articulaire 44 est percée d'un logement sphérique 45 à entrée conique 46 pour recevoir la boule 42.

La tige 40 est de forme généralement cylindrique ou conique et reçoit à sa partie proximale un épaulement 47 destiné à l'appui sur la coupe osseuse radiale. La tige 40 est en outre munie de rainures 48 destinée à faciliter l'ancrage dans le tissus osseux ou le ciment. Le col 41 est incliné par rapport à l'axe de la tige 40 d'un angle β compris entre 0 et 30°.

La tête 44 est terminée par une face concave 49 d'un rayon sensiblement équivalent à celui d'une tête radiale anatomique afin de coopérer avec la portion radiale de la surface épicondylaire 21 de l'élément huméral 2.

On constate que le logement sphérique 45 de la tête 44 débouche dans la partie conique 46 afin d'offrir un débattement suffisant à l'articulation de la boule 42.

On notera que le logement sphérique 45 peut être indifféremment réalisé de manière à retenir ou non la boule 42.

La tête articulaire 44 présente un pourtour extérieur cylindrique coopérant avec la surface articulaire 34 prévue sur le flanc externe de l'embase 32 de l'élément cubital 3.

On note que dans la structure montrée en figure 1 et 2, la tête articulaire 44 est réalisée entièrement en plastique. Cette tête est complètement libre suivant ses trois degrés de liberté en rotation par rapport au centre de la boule 42. Cette configuration permet à la tête articulaire 44 de coopérer efficacement à la fois avec la surface épicondylaire 21 de l'élément huméral 2 et avec la surface artiçulaire 34 de l'élément cubital 3, tout au long du mouvement de flexion de l'avant-bras.

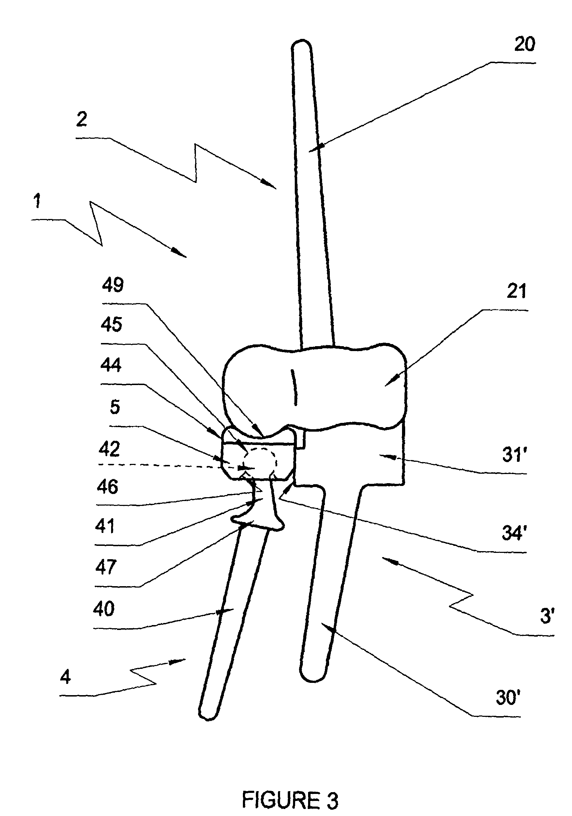

En figure 3, on a représenté une variante de la prothèse totale de coude 1 dont la tête radiale 44 reçoit un cerclage métallique 5 propre à coopérer avec la surface articulaire 34' de l'élément cubital 3' alors réalisée en polyéthylène. En effet, l'élément cubital 3' composé de sa tige 30' et de sa surface articulaire 31' sont réalisés complètement en polyéthylène.

On peut également prévoir que la surface articulaire 31' réalisée en polyéthylène vienne se fixer sur une embase métallique solidaire d'une tige également réalisée en métal. Dans ce cas, la surface articulaire 31' comporte également sur son flanc externe la surface articulaire 34' afin de réaliser l'articulation radio-cubitale.

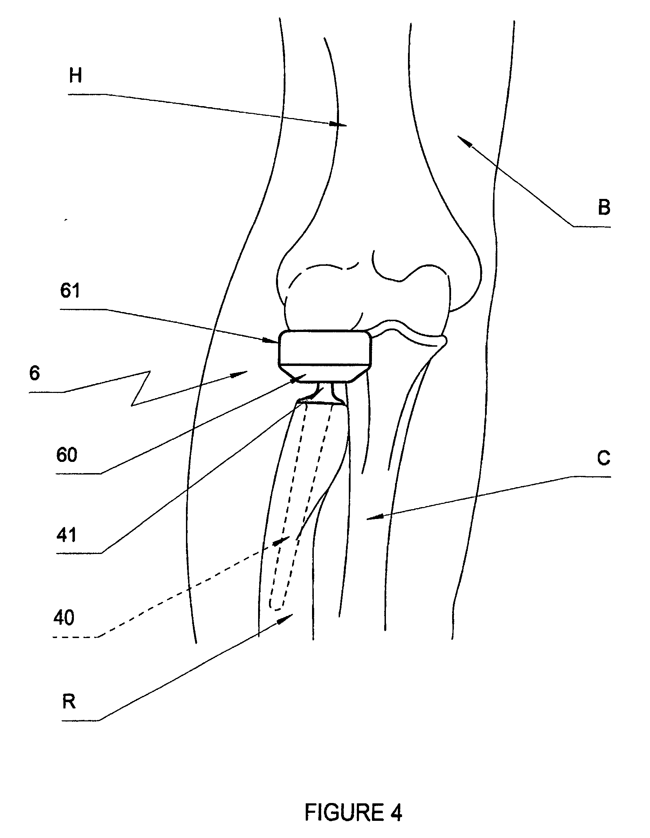

En figure 4, on a montré une seconde variante de la prothèse de coude 1 qui consiste à utiliser uniquement l'élément radial dans une articulation par ailleurs saine. En effet, seul l'élément radial est ancré dans le radius R afin que sa tête articulaire 6, réalisée dans ce cas d'un corps en polyéthylène 60 enveloppé d'une cupule métallique 61 qui coopère à la fois avec les surfaces articulaires anatomiques humérales et cubitale.

La cupule métallique 61 est prévue pour recouvrir le pourtour cylindrique du corps 60 mais également la face supérieure concave qui coopère avec l'articulation humérale saine.

Egalement, le corps 60 présente un logement sphérique identique à celui décrit précédemment afin de recevoir la boule 42 solidaire du col 41 de l'élément radial 4.

On remarque que la tête articulaire 44 et la boule 42 de l'élément radial 4 peuvent être réalisés en tout matériau et plus particulièrement en céramique.

## Revendications

1. Prothèse totale de coude comprenant, un premier élément huméral (2) muni d'une surface articulaire épicondylaire (21), un second élément cubital (3) pourvu d'une surface articulaire (31) et d'un élément radial (4), **caractérisée en ce que** l'élément radial (4) comporte une tige d'ancrage (40) pourvue d'un col (41) qui est incliné d'un angle β compris entre 0 et 30° par rapport à l'axe de ladite tige, ledit col (41) étant solidaire d'une boule (42) sur laquelle vient s'articuler une tête cylindrique (44, 6) comprenant une face supérieure (49) à profil concave qui coopère avec la surface épicondylaire (21) de l'élément huméral (2) et un pourtour cylindrique coopérant avec une surface articulaire externe (34, 34') prévue sur le flanc externe d'une embase (32) de l'élément cubital (3).

2. Prothèse de coude selon la revendication 1 **caractérisée en ce que** la tête articulaire (44) reçoit un cerclage métallique (5) propre à coopérer avec la surface articulaire (34') de l'élément cubital (3') réalisé en polyéthylène.

3. Prothèse de coude selon la revendication 1 **caractérisée en ce que** la tête articulaire (44, 6) comporte un logement sphérique (45) muni d'une partie conique (46) pour recevoir la boule (42) afin de permettre à ladite tête un débattement angulaire.

4. Prothèse de coude selon la revendication 1 **caractérisée en ce que** l'élément radial (4) comporte une tête articulaire (6) pourvue d'un corps en polyéthylène (60) enveloppée d'une cupule métallique (61).

5. Prothèse de coude selon la revendication 1 **caractérisée en ce que** la tête articulaire (44, 6) est complètement libre suivant ses trois degrés de liberté en rotation par rapport au centre de la boule (42).

6. Prothèse de coude selon la revendication 1 **caractérisée en ce que** la tête articulaire (44) et la boule (42) de l'élément radial (4) sont réalisés en céramique.

## Patentansprüche

1. Ellenbogentotalprothese mit einem ersten mit einer Epikondylus-Gelenkfläche (21) versehenen Humerus-Element (2), einem zweiten mit einer Gelenkfläche (31) versehenen Cubitus-Element (3) und einem Radius-Element (4), **dadurch gekennzeichnet, dass** das Radius-Element (4) eine Verankerungsstange (40) mit einem Hals (41) umfasst, der bezüglich der Stangenachse in einem Winkel β zwischen 0 und 30° geneigt und fest mit einer Kugel (42) verbunden ist, an der ein zylindrischer Kopf (44, 6) angelenkt ist, der eine Oberseite (49) mit konkavem Profil, die mit der Epikondylus-Fläche (21) des Humerus-Elements (2) zusammenwirkt, und einen zylindrischen Außenumfang, der mit einer an der Außenflanke einer Sitzfläche (32) des Cubitus-Elements (3) vorgesehenen äußeren Gelenkfläche (34, 34') zusammenwirkt, umfasst.

2. Ellenbogenprothese nach Anspruch 1, **dadurch gekennzeichnet, dass** der Gelenkkopf (44) eine metallische Umreifung (5) erhält, die sich zum Zusammenwirken mit der Gelenkfläche (34') des aus Polyethylen hergestellten Cubitus-Elements (3') eignet.

3. Ellenbogenprothese nach Anspruch 1, **dadurch gekennzeichnet, dass** der Gelenkkopf (44, 6) ein kugelförmiges Lager (45) umfasst, das zur Aufnahme der Kugel (42) mit einem konischen Teil (46) ausgestattet ist, damit der Kopf winkelförmig federn kann.

4. Ellenbogenprothese nach Anspruch 1, **dadurch gekennzeichnet, dass** das Radius-Element (4) einen Gelenkkopf (6) mit einem Körper (60) aus Polyethylen umfasst, der von einem metallischen Becher (61) umhüllt wird.

5. Ellenbogenprothese nach Anspruch 1, **dadurch gekennzeichnet, dass** der Gelenkkopf (44, 6) bei der Drehung bezüglich der Mitte der Kugel (42) gemäß seinen drei Freiheitsgraden völlig frei ist.

6. Ellenbogenprothese nach Anspruch 1, **dadurch gekennzeichnet, dass** der Gelenkkopf (44) und die Kugel (42) des Radius-Elements (4) aus Keramik hergestellt sind.

## Claims

1. Total elbow prosthesis comprising a first, humeral element (2) equipped with an epicondylar articular surface (21), a second, ulnar element (3) provided with an articular surface (31), and a radial element (4), **characterized in that** the radial element (4) includes an anchoring stem (40) provided with a neck (41) which is inclined by an angle β of between 0 and 30° relative to the axis of said stem, said neck (41) being integral with a ball (42) on which a cylindrical head (44, 6) articulates, said cylindrical head (44, 6) comprising an upper face (49) of concave profile which cooperates with the epicondylar surface (21) of the humeral element (2), and a cylindrical circumference cooperating with an outer articular surface (34, 34') provided on the outer flank of a flange (32) of the ulnar element (3).

2. Elbow prosthesis according to Claim 1, **characterized in that** the articular head (44) receives a metal hoop (5) which is able to cooperate with the articular surface (34') of the ulnar element (3') made of polyethylene.

3. Elbow prosthesis according to Claim 1, **characterized in that** the articular head (44, 6) includes a spherical seat (45) equipped with a conical part (46) for receiving the ball (42) in order to permit said head an angular clearance.

4. Elbow prosthesis according to Claim 1, **characterized in that** the radial element (4) includes an articular head (6) provided with a polyethylene body (60) enclosed by a metal cup (61).

5. Elbow prosthesis according to Claim 1, **characterized in that** the articular head (44, 6) is completely free in its three degrees of freedom of rotation relative to the centre of the ball (42).

6. Elbow prosthesis according to Claim 1, **characterized in that** the articular head (44) and the ball (42) of the radial element (4) are made of ceramic.
